# EUROPEAN PATENT APPLICATION

(11) **EP 0 525 367 A1**
(43) Date of publication of application: **03.02.1993**
(21) Application number: 92110272.9
(22) Date of filing: 17.06.1992
(51) Int. Cl.: B01J 27/199, C07C 253/28, C07D 213/84

(54) **Catalyst and process for producing nitrile compounds**

(30) Priority: 10.07.1991 JP 195839/91; 23.07.1991 JP 206411/91
(71) Applicant: MITSUBISHI GAS CHEMICAL COMPANY, INC., Chiyoda-ku, Tokyo (JP)
(72) Inventor: Onda, Yuzi, c/o Niigata Lab., Mitsubishi, Niigata-shi, Niigata-ken (JP); Tsukahara, Kenzo, c/o Niigata Lab., Mitsubishi, Niigata-shi, Niigata-ken (JP); Takahashi, Noriko, c/o Niigata Lab., Mitsubishi, Niigata-shi, Niigata-ken (JP)
(74) Representative: Kraus, Walter, Dr.

(57) **Abstract**

The present invention relates to a catalyst which comprises a molybdenum oxide and a phosphorus oxide prepared from phosphorus molybdic acid or phosphorousmolybdate, a vanadium oxide, a chromium oxide and a boron oxide supported on a silica carrier. This catalyst is suitable for use in the production of nitrile compound by catalytic reaction of a gas mixture containing (1) an alkyl-substituted compound selected from the group of alkyl-substituted aromatic compounds and alkyl-substituted heterocyclic compounds, (2) ammonia, and (3) oxygen or a gas containing molecular oxygen. The present invention further relates to a process for producing the nitrile compound using said catalyst.

## Description

### BACKGROUND OF THE INVENTION

This invention relates to a catalyst suitable for use in producing nitrile compounds by catalytic reaction of a gas mixture containing (1) an alkyl-substituted compound such as alkyl-substituted aromatic compounds and alkyl-substituted heterocyclic compounds, (2) ammonia, and (3) oxygen or a gas containing molecular oxygen over such catalyst, and a process for producing nitrile compounds using the catalyst.

Nitrile compounds are intermediates important in organic chemical industry. For example, phtalonitrile is used as a starting material for producing xylylenediamine useful as synthetic resins, agricultural agents, and curing agents for diisocyanate or epoxy resin. Cyanopyridine is used as a raw material for producing nicotinic acid amide or nicotinic acid which is used in medicines, feed additives, food additives etc.

Hitherto, various process have been proposed for producing aromatic ntiriles by reacting an alkyl-substituted aromatic compound, ammonia and oxygen. Japanese Patent Publication No. 19284/1970 discloses the superior performance of ternary catalyst composed of vanadium, chromium and boron. Further, G.B. Patent 1351523 discloses superiority of the above ternary catalyst which comprises a vanadium oxide, a chromium oxide and a boron oxide at an atomic ratio of 1 : (0.5-2.0) : (0.1-1.2) supported in an amount of 30 - 60% by weight on a silica carrier.

U. S. Patent No. 3,959,339 discloses a high activity of four-component catalyst composed of a vanadium oxide, a chromium oxide, a boron oxide and a phosphorus oxide at an atomic ratio of 1 : (0.5-2.0) : (0.1-1.2) : (0.01-0.3) in a large temperature range. U. S. Patent No. 4,985,581 discloses that reduction with time of selectivity to corresponding aromatic nitrile compounds is very small using a catalyst which composed of a vanadium oxide, a chromium oxide, a molybdenum oxide and a boron oxide at an atomic ratio of 1 : (0.5-2.0) : (0.01-1.2) : (0.01-1.2).

Concerning ammoxidation of alkyl-substituted hetrocyclic compounds to produce corresponding nitrile compounds, U. S. Patent No. 4,963,687 discloses a high yield process for producing a cyanopyridine which comprises reacting the corresponding methylpyridine, ammonia and an oxygen containing gas in the presence of a catalyst comprising a vanadium oxide, a chromium oxide and a boron oxide supported on a silica carrier.

Vapor phase ammoxidation reactions of alkyl-substituted compound generate a large amount of heat of reaction. Therefore, control of reaction temperature is very difficult and fluidized bed type reactor is especially effective for the reaction.

The invention of G. B. patent 1351523 which uses silica as a carrier is an improvement of the invention of Japanese Patent Publication No. 19284/1970, and the catalyst of the former is used in fluidized bed reactors and exhibits excellent performances. The catalyst of U. S. Patent No. 3,959,339 and 4,985,581 exhibits excellent performances also in fluidized bed reactors.

However, these catalyst of the prior art is not enough yield of nitrile compounds, and more improvement of the yield is expected.

### SUMMARY OF THE INVENTION

The inventor has been intensively researched to improve the yield of nitril compound by ammoxidation of alkyl-substituted compound. It has been found that the yield of nitril compound can be improved by using a five-component catalyst which a molybdenum oxide and a phosphorus oxide prepared from a phosphorus molybdic acids or a phosphorusmolybdate, are added to a ternary catalyst composed of vanadium oxide, chromium oxide and boron oxide supported on silica.

Thus, the present invention provides a catalyst suitable for use in producing nitrile compounds by catalytic reaction of a gas mixture containing (1) an alkyl-substituted compound selected from the group of alkyl-substituted aromatic compounds and alkyl-substituted heterocyclic compounds, (2) ammonia, and (3) oxygen or a gas containing molecular oxygen, using a catalyst which comprises a molybdenum oxide and a phosphorus oxide prepared from a phosphorus molybdic acid or a phosphorousmolybdate, a vanadium oxide, a chromium oxide and a boron oxide supported on a silica carrier.

### DETAILED DESCRIPTION OF THE INVENTION

A vanadium oxide, a chromium oxide, a boron oxide may be used as raw materials for the catalyst. Alternatively various compounds, which are readily converted to the corresponding oxides by suitable treatment such as heating when the catalyst is prepared, may be used. These compounds include, for example, ammonium metavanadate, vanadyl sulfate and vanadium salts of organic acids such as oxalic acid and tartaric acid etc., as vanadium raw materials; chromic acid, chromium nitrate, chromium hydroxide, ammonium chromate, ammonium bichromate and chromium salts of organic acids such as oxalic acid and tartaric acid etc., as chromium raw materials; boric acid and ammonium borate as boron raw materials.

A superior performances are found by using a phosphorus molybdic acid or a phosphorusmolybdate as raw materials of molybdenum oxides and phosphorus oxides in this invention. These phosphorus molybdic acid is hetero-polyoxides usually synthesised by solution-heating process. A phosphorus molybdic acids such as H₃(PMo₁₂O₄₀), H₇-(PMo₁₁o₃₉), H₆(P₂Mo₁₈O₆₂), a phosphorusmolybdate such as ammonium phosphomolybdate (NH₄)-₃(PMo,2040) can be used. A superior performances may not be attained when a catalyst is prepared using a ammonium para-molybdate as raw materials of molybdenum oxides and a phosphoric acid as phosphorus oxides, without using a phosphorus molybdic acid or a phosphorusmolybdate as raw materials of molybdenum oxides and phosphorus oxides.

The atomic ratio of vanadium : chromium : boron : molybdenum : phosphorus which are catalyst component of present invention is preferably 1 : (0.5-2.0) : (0.01-1.2) : (0.01-1.2) : (0.001-0.1). The yield of the nitril compound may be decreased when the atomic ratio is out side of this range.

As the silica which supports these catalyst components, for example, silica gel, colloidal silica and anhydrous silica which are disclosed in "Kagaku Binran (Handbook of Chemistry), Section of Applied Chemistry I", page 256-258 (published from Maruzen Co. in 1986) may be used. Total percentage of the catalyst component oxide in the catalyst is 20-80% by weight, preferably 30-60% by weight (the oxide being expressed as V₂0₅, Cr₂0₃, B₂0₃, MoOs and P₂0s, respectively).

The present catalyst can be prepared by known methods. For example, aqueous phosphorus molybdic acid solution and aqueous boric acid solution are added to a solution of vanadium oxalate and chromium oxalate and then a silica sol is added thereto obtain a slurry mixture. In this case, if necessary, a dissolving aid for boric acid is used. The dissolving aid for boric acid includes polyhydric alcoholes, a -monooxycarboxylic acids, and dioxycarboxylic acids.

In the case of a fluidized bed catalyst, the resulting mixture is spray dried and, if necessary, is further dried at 110 - 150°C and then calcined. In the case of a fixed bed catalyst, the mixture is evaporated to dryness and then is calcined. Calcination is carried out at 400 - 700 ° C , preferably 450 - 650 °C for a few hours or more while passing air. If prior to this calcination a preliminary calcination is carried out at 200 - 400 ° C, more favorable results can be obtained.

The alkyl-substituted aromatic compounds used as a starting material in the present invention include, for example, toluene, ethylbenzene, poly- methylbenzene, (such as xylene, mesitylene, cymene, and durene), diethylbenzene and methylnaphthalene, dimethylnaphthalene. The alkyl-substituted heterocyclic compounds include methylpyridine, ethylpyridine, dimethylpyridine and methylquinoline etc..

A suitable concentration of the alkyl-substituted compound in the gas mixture is 0.5 - 5 vol % when air is used as the oxygen source.

Amount of ammonia used may be at least the theoretical amount (1 mol of ammonia per 1 mol of alkyl group). The higher molar ratio of ammonia / alkyl-substituted compound in the gas mixture is advantageous for improving yield of nitrile from the alkyl-substituted compound, but in view of the necessity to recover unreacted ammonia, amount of ammonia is the thoretical amount or more, preferably about 2 - 10 times as much as the theoretical amount.

Usually, air is used as oxygen source, and nitrogen, carbon dioxide or steam can be used as an inert diluents. Amount of oxygen to fed is at least 1.5 times as mush as the theoretical amount and preferably 2 - 50 times as much as the theoretical amount.

Temperature of the reaction can be in a wide range of 300 - 500 °C, but preferably 330 - 470 ° C . When the temperature is lower than 300 ° C , conversion of the raw material compound is low and when higher than 500 ° C , production of carbon dioxide and hydrogen cyanide is increased, resulting in reduction in the yield of nitrile. Reaction temperature for obtaining the maximum yield of nitrile depends on the kind of alkyl-substituted compound, concentration of raw materials, contact time and calcination conditions for catalyst. So it is preferred to optionally select the reaction temperature within the above range depending on the conditions.

Contact time of the gas mixture with the catalyst may be varied over a wide range but is preferably 0.5 - 30 seconds.

The reaction of the present invention is usually carried out under atmospheric pressure, but can be carried out under an elevated pressure or a reduced pressure.

Collection of reaction product can be effected by any suitable methods, for example, by cooling to a temperature enough to precipitate the product or by washing the reaction product gas with water or other suitable solvent.

As mentioned above, since the reaction of the present invention involves vigorous generation of heat, it is advantageous to carry out the reaction in a fluidized bed or a moving bed for removal of heat of reaction to prevent partial heating, although the characteristics of the catalyst can be exhibited and the excellent performance can be maintained even if the reaction is carried out in a fixed bed.

According to the process of the present invention which comprises carrying out the catalyst reaction of a gas mixture containing an alkyl-substituted compound, ammonia and oxygen or a gas containing molecular oxygen using a catalyst which comprises a molybdenum oxide and a phosphorus oxide prepared from phosphorus molbdic acid or phosphorus-molybdate, a vanadium oxide, a chromium oxide and a boron oxide supported on silica carrier thereby to produce nitrile compounds, excellent high yield is obtained as shown by the following examples.

Although a catalyst composed of a vanadium oxide, a chromium oxide, a boron and a phosphorous oxide is disclosed in U. S. Patent No. 3,959,339, and a catalyst composed of a vanadium oxide, a chromium oxide, a boron and a molybdenum oxide is disclosed in U. S. Patent No. 4,985,581 as aforementioned. The use of a phosphorus molbdic acid or a phosphorusmolybdate for a molybdenum oxide as raw material is not disclosed yet, and a catalyst composed of a vanadium oxide, a chromium oxide, a boron, a molybdenum oxide and a phosphorus oxide is a new catalyst for these ammoxidation reaction.

A excellent high yield is obtained only in the use of a phosphorus molbdic acid or a phosphorus- molybdate for a molybdenum oxide and a phosphorus oxide. This excellent performance is not obtained in the use of a molybdic acid or molybdate and a phosphoric acid as showen by Comparative Example 1. So the present invention is a new invention, and is not obvious from above- mentioned U. S. Patent etc..

### DESCRIPTION OF THE PREFERRED EMBODIMENTS

The present invention will be explained in more detail by the following example and comparable examples. In these examples, wt% means % by weight.

### Comparable Example 1

### (Preparation of catalyst)

500 ml of water was added to 229 g of vanadium pentaoxide, V₂O₅, followed by heating to 80 - 90 °C , 477 g of oxalic acid, (COOH)₂2H₂0, was added with well stirring to obtain a vanadyl oxalate solution. Then, 400 ml of water was added to 963 g of oxalic acid, followed by heating to 50 - 60 ° C , a solution of 252 g of chromic anhydride, Cr0₃, in 200 ml of water was added with well stirring to obtain a chromic oxalate solution. The chromic oxalate solution was mixed with the vanadyl oxalate solution at 50 - 60 ° C to obtain vanadium-chromium solution.

Separately, 300 ml of water was added to 44 g of ammonium paramolybdate, (NH₄)₆MoO₂₄' 4H₂0, then 3 g of 85 wt % of phosphoric acid, H3PO4 was added and mixed with well stirring to obtain a molibdenium-phosphoric acid solution.

The vanadium-chromium solution was added with the molibdenium-phosphoric acid solution, to which 1667 g of a 30 wt% aqueous silica sol was added.

To this slurry solution was added 78 g of boric acid, H₃BO₃, then this slurry was well mixed and concentrated until amount of the liquid reached about 3800 g.

This catalyst solution was spray dried with an inlet gas temperature of 250 ° C and an outlet gas temperature of 130 °C . The thus spray dried catalyst was dried in a drier of 130 ° C for 12 hours. Then, the catalyst was preliminary calcined at 400 ° C for 0.5 hour and thereafter calcined at 550 °C for 8 hours while passing air. This catalyst had an atomic ratio V : Cr : B : Mo : P of 1 : 1 : 0.5 : 0.1 : 0.01 and an oxide concentration of 50 wt%.

### (Test for catalyst performance)

A 40 ml of the resulting catalyst was packed in a reactor having an inner diameter of 23 mm which was heated in a molten salt bath, a preheated gas mixture consisting of 3.0 vol % of m-xylene, 21.0 vol % of ammonia and 76 vol % of air was contacted with the catalyst at SV of 750 Hr-¹ and at 370 ° C which gives the maximum yield of isophthalonitrile. The yield of isophthalonitrile and m-tolunitril were 76.9 mol% and 4.9 mol % based on m-xylene respectively. Selectivity of the catalyst for isophthalonitrile to the reacted m-xylene was 79.2 mol %.

### Example 1

### (Preparation of catalyst)

500 ml of water was added to 229 g of vanadium pentaoxide, V₂O₅, followed by heating to 80 - 90 °C , 477 g of oxalic acid, (COOH)₂2H₂0, was added with well stirring to obtain a vanadyl oxalate solution. Then, 400 ml of water was added to 963 g of oxalic acid, followed by heating to 50 - 60 ° C , a solution of 252 g of chromic anhydride, Cr0₃, in 200 ml of water was added with well stirring to obtain a chromic oxalate solution. The chromic oxalate solution was mixed with the vanadyl oxalate solution at 50 - 60°C to obtain vanadium-chromium solution.

To this solution were added a solution of 49.6 g of phosphorus molybdic acid, H₃(PMo₁₂O₄₀)' 30H₂0, in 100 ml of water and furthermore, 1667 g of a 30 wt % aqueous silica sol. To this slurry solution was added 78 g of boric acid, H₃BO₃, then this slurry was well mixed and concentrated until amount of the liquid reached about 3800 g.

This catalyst solution was spray dried with an inlet gas temperature of 250 ° C and an outlet gas temperature of 130 °C . The thus spray dried catalyst was dried in a drier of 130 ° C for 12 hours. Then, the catalyst was preliminary calcined at 400 ° C for 0.5 hour and thereafter calcined at 550 °C for 8 hours while passing air. This catalyst had an atomic ratio V : Cr : B : Mo : P of 1 : 1 : 0.5 : 0.1 : 0.01 and an oxide concentration of 50 wt %.

### (Test for catalyst performance)

In the same manner as in Comparable Example 1, the resulting catalyst was examined on activity.

A gas mixture consisting of 3.0 vol% of m-xylene, 21.0 vol% of ammonia and 76 vol% of air was fluid contacted with the catalyst at Sin of 750 Hr⁻¹ and at 390 ° C which is a temperature to give maximum yield of isophthalonitrile. The yield of isophthalonitrile and m-tolunitril were 84.2 mol% and 2.2 mol% based on m-xylene respectively. Selectivity of the catalyst for isophthalonitrile to the reacted m-xylene was 84.7 mol%.

### Example 2

A catalyst having an atomic ratio of V : Cr : B : Mo : P = 1 : 1 : 0.5 : 0.2 : 0.02 was prepared in the same manner as in Example 1 and activity of this catalyst was examined.

A gas mixture consisting of 3.0 vol% of m-xylene, 21.0 vol% of ammonia and 76 vol% of air was fluid contacted with the catalyst at SV of 750 Hr⁻¹ and at 410 °C which is a temperature to give maximum yield of isophthalonitrile. The yield of isophthalonitrile and m-tolunitril were 83.0 mol% and 2.8 mol% based on m-xylene respectively. Selectivity of the catalyst for isophthalonitrile to the reacted m-xylene was 83.4 mol%.

### Example 3

In place of phophorous molbdic acid in Example 1. using ammonium phoshmolybdate, (NH₄)₃-(PMo₁₂O₄₀)' 3H₂O, a catalyst having an atomic ratio of V : Cr : B : Mo : P = 1 : 1 : 0.5 : 0.1 : 0.01 was prepared in the same manner as in

### Example 1 and activity of this catalyst was examined.

A gas mixture consisting of 3.0 vol% of m-xylene, 21.0 vol% of ammonia and 76 vol% of air was fluid contacted with the catalyst at SV of 750 Hr⁻¹ and at 390 ° C which is a temperature to give maximum yield of isophthalonitrile. The yield of isophthalonitrile and m-tolunitril were 84.1 mol% and 2.1 mol% based on m-xylene respectively. Selectivity of the catalyst for isophthalonitrile to the reacted m-xylene was 84.5 mol %.

### Example 4

A catalyst in Example 1 was tested on activity in the same manner as in Example 1 except that p-xylene was used in place of m-xylene.

A gas mixture consisting of 3.2 vol% of p-xylene, 19.5 vol% of ammonia and 77.3 vol% of air was fluid contacted with the catalyst at Sin of 800 Hr⁻¹ and at 400 ° C which is a temperature to give maximum yield of terephthalonitrile. The yield of terephthalonitrile and p-tolunitrile were 84.8 mol % and 1.9 mol % based on p-xylene, respectively. Selectivity of the catalyst for terephthalonitrile to the reacted p-xylene was 85.2 mol %.

### Example 5

A catalyst in Example 1 was tested on activity in the same manner as in Example 1 except that tolene was used in place of m-xylene.

A gas mixture consisting of 5.1 vol% of tolene, 25.0 vol% of ammonia and 69.9 vol% of air was fluid contacted with the catalyst at SV of 840 Hr-¹ and at 410 °C which is a temperature to give maximum yield of benzonitrile. The yield of benzonitrile was 84.5 mol% based on tolene. Selectivity of the catalyst for benzonitrile to the reacted tolene was 84.9 mol%.

### Example 6

A catalyst in Example 1 was tested on activity in the same manner as in Example 1 except that 3-methyl-pyridne was used in place of m-xylene.

A gas mixture consisting of 3.0 vol% of 3-methylpyridne, 12.0 vol% of ammonia and 85.0 vol% of air was contacted with the catalyst at sin of 750 Hr -¹ and at 390 ° C which is a temperature to give maximum yield of 3-cyanopyridne. The yield of 3-cyanopyridne was 93.7 mol% based on 3-methyl-pyridne. Selectivity of the catalyst for 3-cyanopyridne to the reacted 3-methyl-pyridne was 94.2 mol%.

## Claims

1. A catalyst suitable for use in producing nitrile compound by catalytic reaction of a gas mixture containing (1) an alkyl-substituted compound selected from the group of alkyl-substituted aromatic compounds and alkyl-substituted heterocyclic compounds, (2) ammonia, and (3) oxygen or a gas containing molecular oxygen using a catalyst which comprises a molybdenum oxide and a phosphorus oxide prepared from phosphorus molybdic acid or phosphorous-molybdate, a vanadium oxide, a chromium oxide and a boron oxide supported on a silica carrier.

2. A catalyst according to claim 1 wherein the atomic ratio of vanadium : chromium : boron : molybdenum : phosphine is 1 : 0.5-2.0 : 0.01-1.2 : 0.01-1.2 : 0.001-0.1.

3. A catalyst according to claim 1 wherein the proportion of the vanadium oxide, the chromium oxide, the boron oxide, molybdenum oxide and the phosphorus oxide in the catalyst is 20 - 80 % by weight.

4. A process for producing nitrile compound which comprises subjecting to a catalytic reaction a gas mixture containing (1) an alkyl-substituted compound selected from the group of alkyl-substituted aromatic compounds and alkyl-substituted heterocyclic compounds, (2) ammonia, and (3) oxygen or a gas containing molecular oxygen using a catalyst which comprises a molybdenum oxide and a phosphorus oxide prepared from phosphorus molybdic acid or phosphorousmolybdate, a vanadium oxide, a chromium oxide and a boron oxide supported on a silica carrier.

5. A process according to claim 4 wherein the atomic ratio of vanadium : chromium : boron : molybdenum : phosphorus is 1 : 0.5-2.0 : 0.01-1.2 : 0.01-1 .2 : 0.001-0.1

6. A process according to claim 4 wherein the proportion of the vanadium oxide, the chromium oxide, the boron oxide, molybdenum oxide and the phosphorus oxide in the catalyst is 20 - 80 % by weight.

7. A process according to claim 4 wherein the alkyl-substituted aromatic compounds is toluene, ethylbenzene, xylene, mesitylene, cymene, durene, diethylbenzene or methylnaphthalene.

8. A process according to claim 4 wherein the alkyl-substituted heterocyclic compounds is methylpyridine, ethylpyridine, dimethylpyridine or methylquinoline.

9. A process according to claim 4 wherein the gas miture contains ammonia in an amount not less than the theoretical amount.

10. A process according to claim 4 wherein the reaction temperatue is 300 - 500 ° C .

11. A process according to claim 4 wherein the gas containg molecular oxygen is air.

12. A process according to claim 4 wherein the amount of oxygen present in the gas mixture is at least 1.5 times of the theoretical amount.

13. A process according to claim 4 wherein the reaction is carried out in a fluidized bed catalyst.
